# EUROPÄISCHE PATENTSCHRIFT

(11) **EP 2 238 950 B1**
(45) Veröffentlichungstag und Bekanntmachung des Hinweises auf die Patenterteilung: **28.11.2012**
(21) Anmeldenummer: 10000517.2
(22) Anmeldetag: 20.01.2010
(51) Int. Cl.: A61F 2/46

(54) **Chirurgisches Instrument**
Surgical instrument
Instrument chirurgical

(30) Priorität: 07.04.2009 DE 202009004686 U
(43) Veröffentlichungstag der Anmeldung: 13.10.2010
(73) Patentinhaber: Weber Instrumente GmbH & Co. KG, 78576 Emmingen-Liptingen (DE)
(72) Erfinder: Weber, Helmut, 78576 Emmingen-Liptingen (DE)
(74) Vertreter: Modrow, Stephanie

(56) Entgegenhaltungen:
- US-A1- 2004 059 271
- US-A1- 2004 088 054
- US-A1- 2007 055 378
- US-A1- 2007 282 441

## Beschreibung

Die Erfindung betrifft ein chirurgisches Instrument gemäß dem Oberbegriff des Patentanspruchs 1.

Bekannt sind chirurgische Instrumente, insbesondere zum Halten eines Implantats, welche ein Rohr und eine koaxial in dem Rohr angeordnete Stange aufweisen. An dem distalen Ende der Stange ist ein Arbeitsende angeordnet, wobei die Stange mittels einer Verstellvorrichtung um ihre Längsachse drehbar in dem Rohr angeordnet ist. Das Arbeitsende kann bspw. ein Gewinde aufweisen, über welches ein Implantat an der Stange befestigt werden kann, so dass mit Hilfe des Instruments das Implantat während der Operation in den Patienten eingesetzt werden kann. Nach dem Einsetzen kann durch Abschrauben der Stange die Verbindung zwischen dem Instrument und dem Implantat gelöst und das Instrument entfernt werden.

Bei den bekannten Instrumenten ist die Stange als Spindel ausgeführt, welche in ein in dem Rohr angeordnetes Innengewinde eingreift und mit Hilfe einer am proximalen Ende der Stange angeordneten Verstellmutter um die Längsachse drehbar in dem Rohr angeordnet ist.

Beim Einsetzen des Implantats ist es häufig vonnöten, auf das proximale Ende des Instruments Schläge auszuüben, um das Implantat an die richtige Position setzen zu können. Wird bei der bekannten Vorrichtung mit einem Hammer auf das proximale Ende des Instruments geschlagen, überträgt sich die Kraft direkt auf die Verbindung zwischen der Stange und dem Implantat, insbesondere auf das Gewinde, was dabei beschädigt werden kann.

Als Stand der Technik werden die US 2004/059271 A1, US 2004/088054 A1, US 2007/055378 A1 und US 2007/282441 A1 genannt.

Die Aufgabe der Erfindung besteht daher darin, ein chirurgisches Instrument, insbesondere zum Halten des Implantats, bereit zu stellen, welches diesen Nachteil beseitigt.

Die Aufgabe der Erfindung wird gelöst durch ein chirurgisches Instrument mit den Merkmalen des Patentanspruchs 1.

Vorteilhafte Ausgestaltungen und Weiterbildungen sind in den Unteransprüchen angegeben.

Das erfindungsgemäße chirurgische Instrument, insbesondere zum Halten eines Implantats, mit einem Rohr, welches ein distales Ende und ein proximales Ende aufweist, und einer koaxial in dem Rohr angeordneten Stange, welche ein distales und ein proximales Ende aufweist, wobei an dem distalen Ende der Stange in Arbeitsende angeordnet ist, und wobei die Stange mittels einer Verstellvorrichtung um ihre Längsachse drehbar in dem Rohr angeordnet ist, zeichnet sich dadurch aus, dass das proximale Ende der Stange innerhalb des Rohres angeordnet ist. Das proximalen Ende des chirurgischen Instruments wird somit nicht wie bei den gemäß dem Stand der Technik bekannten chirurgischen Instrumenten durch das proximale Ende der Stange, sondern durch das proximale Ende des Rohres gebildet. Wird mit einem Hammer auf das proximale Ende des chirurgischen Instruments geschlagen, wird somit auf das proximale Ende des Rohres und nicht mehr auf das proximale Ende der Stange geschlagen, so dass die Kraft der Schläge nicht auf die Stange und somit nicht auf das Arbeitsende der Stange, insbesondere die Gewindeverbindung der Stange und einem Implantat, wirkt. Das Arbeitsende, insbesondere ein als Gewinde ausgebildetes Arbeitsende, wird somit vor Schädigung geschützt.

Gemäß einer besonders bevorzugten Ausführungsform der Erfindung ist die Verstellvorrichtung zwischen dem distalen und dem proximalen Ende des Rohres angeordnet. Insbesondere weist das Rohr einen distalen Rohrabschnitt und einen proximalen Rohrabschnitt auf, wobei die Verstellvorrichtung zwischen dem distalen Rohrabschnitt und dem proximalen Rohrabschnitt angeordnet ist. Die Verstellvorrichtung wird somit von dem proximalen Ende des Instruments weg verlagert.

Vorteilhafterweise ist an dem Rohr ein Handgriff angeordnet, um die Handhabung des chirurgischen Instruments angenehmer für den Benutzer zu gestalten.

Gemäß einer besonders bevorzugten Ausführungsform der Erfindung ist die Verstellvorrichtung axial anschließend an das distale Ende des Handgriffs angeordnet, so dass eine Betätigung der Verstellvorrichtung bei Halten des Handgriffs in einer Hand durch Daumen und Zeigefinger der gleichen Hand ermöglicht wird und somit eine einhändige Benutzung des chirurgischen Instruments möglich ist.

Gemäß einer besonders bevorzugten Ausführungsform der Erfindung ist die Verstellvorrichtung als Zahnradgetriebe ausgebildet. Einerseits ermöglicht ein Zahnradgetriebe insbesondere bei entsprechender Übersetzung eine einfache Betätigung der Verstellvorrichtung. Andererseits ermöglicht ein Zahnradgetriebe eine Entkopplung von Rohr und Stange in axialer Richtung, so dass Schläge auf das proximale Ende des Rohres nicht auf die Stange und das daran angeordnete Arbeitsende übertragen werden.

In einer Ausführungsform der Erfindung ist die Verstellvorrichtung als Planetengetriebe ausgebildet, was konstruktiv besonders einfach zu realisieren ist. Ein Planetengetriebe weist jedoch den Nachteil auf, dass der Antrieb und der Abtrieb gegensinnige Drehrichtungen aufweisen.

Gemäß einer besonders bevorzugten Ausführungsform der Erfindung weist daher die Verstellvorrichtung ein Betätigungselement auf, wobei das Betätigungselement und die Stange gleiche Drehrichtung aufweisen. Dies vereinfacht die Benutzung des chirurgischen Instruments.

Erfindungsgemäß weist die Verstellvorrichtung eine Betätigungselement auf, welches als Zahnradring mit einer Innenverzahnung ausgebildet ist, wobei innerhalb des Betätigungselements ein Übersetzungselement angeordnet ist, welches als Zahnradring mit einer Außenverzahnung, welche mit der Innenverzahnung des Betätigungselements in Eingriff steht, und einer Innenverzahnung ausgebildet ist, und wobei innerhalb des Übersetzungselements ein Übertragungselement angeordnet ist, welches als Zahnradring mit einer Außenverzahnung, welche mit der Innenverzahnung des Übersetzungselements in Eingriff steht, ausgebildet ist. Auf diese Weise wird insbesondere ermöglicht, dass das Betätigungselement und das Übertragungselement gleiche Drehrichtung aufweisen.

Gemäß einer bevorzugten Ausführungsform der Erfindung ist die Drehachse des Betätigungselements und die Drehachse des Übertragungselements identisch, während die Drehachse des Übersetzungselements parallel versetzt zu den Drehachsen des Betätigungselements und des Übertragungselements angeordnet ist, wodurch eine symmetrische Anordnung, insbesondere eine koaxiale Anordnung der des Betätigungselements und des Übertragungselements und somit insbesondere der Stange und des Rohres, ermöglicht wird.

Vorzugsweise ist das Übertragungselement mit der Stange drehfest verbunden. Die drehfeste Verbindung kann bspw. durch eine einstückige Ausbildung von Stange und Übertragungselement erreicht werden.

Gemäß einer bevorzugten, alternativen Ausführungsform der Erfindung weist das Übertragungselement jedoch eine Durchgangsöffnung mit einer unrunden Innenkontur auf, in welche die Stange mit einem Abschnitt, der eine entsprechende Außenkontur aufweist, im Wesentlichen formschlüssig einsetzbar ist. Dies ermöglicht insbesondere in gewissem Maße eine axiale Bewegung zwischen der Stange und dem Übertragungselement und somit der Stange und der Verstellvorrichtung.

Vorzugsweise entspricht der Außendurchmesser der Verstellvorrichtung, insbesondere des Betätigungselements, im Wesentlichen dem Außendurchmesser des Rohres und/oder des Handgriffs, um die äußeren Dimensionen des chirurgischen Instruments möglichst gering zu halten.

Vorzugsweise ist das distale Ende der Stange als Gewinde, Sechskant, Sechsrund, Zahnrad, Kegelrad oder Schnecke ausgebildet, um auf diese Weise vielfältige Anwendungsmöglichkeiten des chirurgischen Instruments zu bieten, und es insbesondere zu ermöglichen, ein Implantat zu spannen, zu halten, zu lösen, einzubringen, zu manipulieren oder auszubringen.

Ein Ausführungsbeispiel wird anhand der nachfolgenden Figuren ausführlich erläutert. Es zeigt:

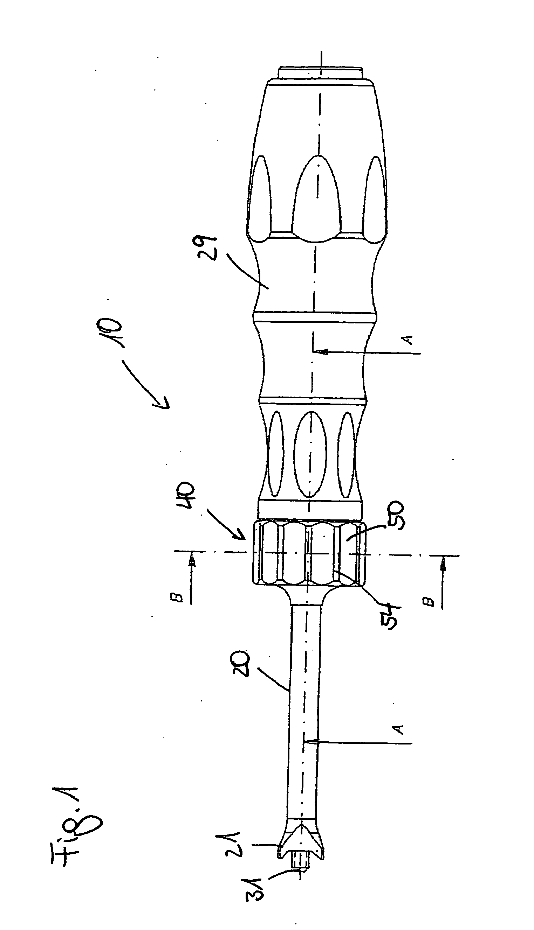

- Fig. 1: eine Seitenansicht eines Ausführungsbeispiels eines chirurgischen Instruments,

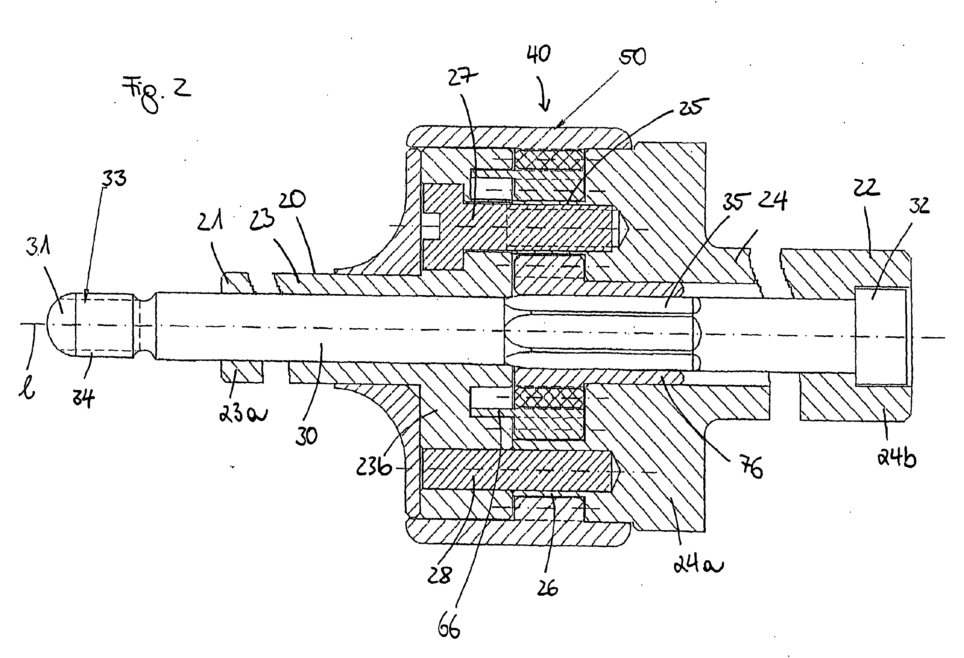

- Fig. 2: einen Längsschnitt entlang der Linie A-A aus Fig. 1 mit der Stange in nichtgeschnittener Darstellung und

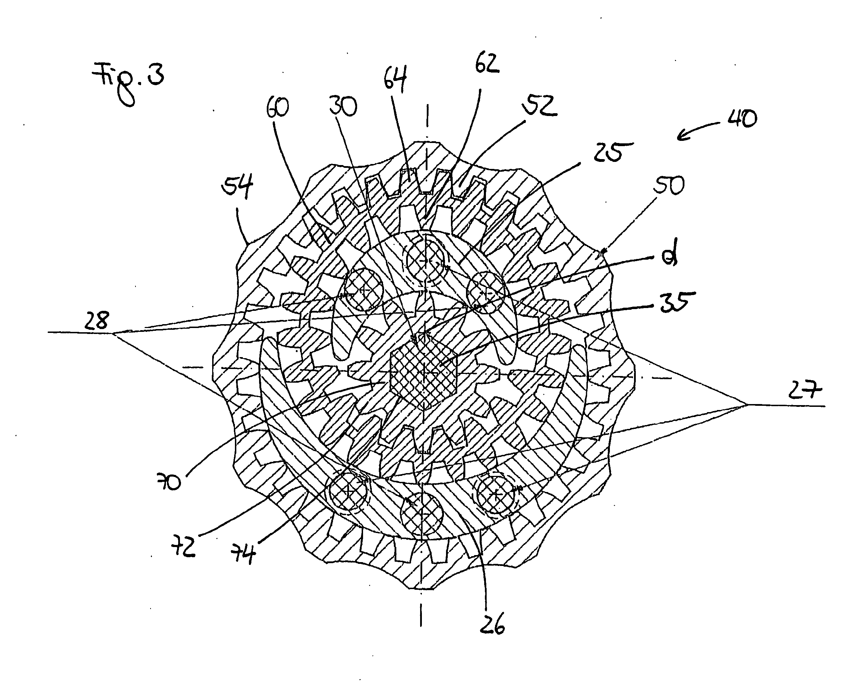

- Fig. 3: einen Querschnitt entlang der Linie B-B aus Fig. 1.

In den Figuren ist ein chirurgisches Instrument 10 dargestellt, welches insbesondere zum Halten, Spannen, Lösen, Einbringen, Manipulieren und/oder Ausbringen eines Implantats, insbesondere eines sogenannten Cages, verwendet wird.

Das Instrument 10 weist ein Rohr 20 mit einem distalen Ende 21 und einem proximalen Ende 22 auf, wobei das Rohr 20 einen distalen Rohrabschnitt 23 mit einem ersten Ende 23a und einem zweiten Ende 23b und einen proximalen Rohrabschnitt 24 mit einem ersten Ende 24a und einem zweiten Ende 24b aufweist. Die einander zugewandten Enden 23b, 24a der Rohrabschnitte 23, 24 weisen einen Bund auf, in welchem sich der Außendurchmesser des Rohres stufenartig erweitert. An der Stirnfläche des ersten Endes 24a des proximalen Rohrabschnitts 24 sind zwei halbmondförmige Vorsprünge 25, 26 angeordnet, welche sich in axialer Richtung erstrecken und die unter anderem als Abstandshalter zwischen dem zweiten Ende 23b des distalen Rohrabschnitts und dem ersten Ende 24a des proximalen Rohrabschnitts 24 dienen. Das zweite Ende 23b des distalen Rohrabschnitts 23 und das erste Ende 24a des proximalen Rohrabschnitts 24 sind über drei Schrauben 27 miteinander verschraubt, wobei zusätzlich drei Stifte 28 als Positionierhilfen dienen. Die Schrauben 27 und die Stifte 28 sind insbesondere in axialer Richtung angeordnet.

Im Anschluss an den am ersten Ende 24a des proximalen Rohrabschnitts 24 angeordneten Bund ist ein sich in proximaler Richtung erstreckender Handgriff 29 angeordnet, der insbesondere aus einem Kunststoff, insbesondere einem elastischen Kunststoff, gefertigt ist und bspw. direkt auf den proximalen Rohrabschnitt 24 aufgespritzt werden kann.

Durch das Rohr 20 ist koaxial eine Stange 30 mit einem distalen Ende 31 und einem proximalen Ende 32 geführt, welche eine Längsachse 1 aufweist, um welche die Stange 30 innerhalb des Rohres 20 verdrehbar ist. An dem distalen Ende 31 der Stange 30 ist ein Arbeitsende 33 angeordnet, welches je nach Anwendungszweck verschiedene Ausgestaltungen haben kann, bspw. als Sechskant, Sechsrund, Zahnrad, Kegelrad oder Schnecke ausgebildet sein kann und im vorliegenden Ausführungsbeispiel als Gewinde 34 ausgebildet ist. Das Gewinde 34 kann durch Drehung der Stange 30 um die Längsachse 1 bspw. in ein entsprechendes Gewinde eines Implantats eingeschraubt werden, um auf diese Weise das Implantat an dem Instrument 10 befestigen zu können und entsprechend handhaben zu können, bspw. um das Implantat in eine gewünschte Position im Patienten zu bewegen, dort einzubringen, wieder zu entfernen oder auf sonstige Weise zu handhaben.

Die Stange 30 weist einen axialen Abschnitt 35 mit einem unrunden Querschnitt, vorliegend insbesondere der Kontur eines Sechskants, auf. Dieser Abschnitt 35 liegt insbesondere im Verbindungsbereich zwischen dem distalen Rohrabschnitt 23 und dem proximalen Rohrabschnitt 24.

In dem Zwischenbereich zwischen den Stirnseiten des zweiten Endes 23b des distalen Rohrabschnitts 23 und des ersten Endes 24a des proximalen Rohrabschnitts 24 ist eine Verstellvorrichtung 40 angeordnet, über welche die Stange 30 um ihre Längsachse 1 drehbar ist. Die axiale Lagerung der Verstellvorrichtung 40 erfolgt somit durch die Stirnseite des zweiten Endes 23b des distalen Rohrabschnitts 23 sowie die Stirnseite des ersten Endes 24a des proximalen Rohrabschnitts 24.

Die Verstellvorrichtung 40 weist ein Betätigungselement 50 auf, welches im Wesentlichen als hohlzylindrisches Rohr ausgebildet ist, das auf seiner Innenseite eine umlaufende Innenverzahnung 52 aufweist, welche insbesondere nicht über die gesamte Länge des Betätigungselements 50, sondern vorzugsweise in Längsrichtung nur abschnittsweise angeordnet ist. Das Betätigungselement 50 ist somit insbesondere als Zahnradring ausgebildet. Die Innenverzahnung 52 erstreckt sich über eine Länge des Betätigungselements 50, die dem Abstand zwischen den Stirnseiten des zweiten Endes 23b des distalen Rohrabschnitts 23 und des ersten Endes 24a des proximalen Rohrabschnitts 24 entspricht und zwischen diesen Enden 23b, 24a in den Zwischenraum hineinragt, während die glatten Abschnitte der Innenfläche des Betätigungselements 50 auf dem Außenumfang des zweiten Endes 23b des distalen Rohrabschnitts 23 und des ersten Endes 24a des proximalen Rohrabschnitts 24 anliegen, wodurch insbesondere eine radiale Lagerung des Betätigungselements 50 erfolgt. Das Betätigungselement 50 ist somit koaxial zu dem Rohr 20 angeordnet. Die Außenseite des Betätigungselements 50 weist ein Profil 54, bspw. in Form von Längsrippen auf, um auf diese Weise ein Abrutschen der Finger des Benutzers zu vermeiden.

Die Verstellvorrichtung 40 und insbesondere das Betätigungselement 50 sind axial im Wesentlichen angrenzend an das distale Ende des Handgriffs 29 angeordnet, so dass die Verstellvorrichtung 40 und insbesondere das Betätigungselement 50 mit dem Daumen und den Zeigefingern der Hand, die den Handgriff 29 hält, betätigt werden kann.

Innerhalb des Betätigungselements 50 ist ein Übersetzungselement 60 angeordnet, welches im Wesentlichen als Hohlzylinder ausgebildet ist und auf der Außenfläche eine Außenverzahnung 64 und auf der Innenfläche eine Innenverzahnung 62 aufweist. Das Übersetzungselement 60 ist somit als Zahnradring ausgebildet. Die Außenverzahnung 64 des Übersetzungselements 60 steht mit der Innenverzahnung 52 des Betätigungselements 50 in Eingriff, so dass bei Drehen das Betätigungselement 50 auch das Übersetzungselement 60 gedreht wird.

Das Betätigungselement 50 lässt sich um seine Längsachse drehen, die im Wesentlichen, abgesehen von Toleranzen, mit der Längsachse 1 der Stange 30 zusammenfällt.

Das Übersetzungselement 60 ist exzentrisch relativ zu dem Betätigungselement 50 angeordnet. Das Übersetzungselement 60 dreht sich um eine Drehachse d, die parallel versetzt zur Längsachse 1 der Stange 30 und somit zur Drehachse des Betätigungselements 50 verläuft.

Eine radiale Lagerung des Übersetzungselements 60 erfolgt über einen im Bereich zwischen der Innenverzahnung 62 und der Außenverzahnung 64 an dem Zahnradring angeordneten, sich in axialer Richtung erstreckenden Steg 66, der in eine umlaufende, sich in axialer Richtung erstreckende Nut 23c, welche in der Stirnseite des zweiten Endes 23b des distalen Rohrabschnitts 23 angeordnet ist, eingreift.

Der Außendurchmesser des Übersetzungselements 60 ist kleiner als der Durchmesser der Innenverzahnung 52 des Betätigungselements 50. In den Zwischenraum zwischen dem Übersetzungselement 60 und dem Betätigungselement 50 greift der halbmondförmige Vorsprung 26 ein.

Im Inneren des Übersetzungselements 60 ist ein Übertragungselement 70 angeordnet, welches eine Außenverzahnung 72 aufweist und eine Durchgangsöffnung 74 aufweist. Die Außenverzahnung 72 des Übertragungselements 70 steht mit der Innenverzahnung 62 des Übersetzungselements 60 in Eingriff.

Der Außendurchmesser des Übertragungselements 70 ist kleiner als der Durchmesser der Innenverzahnung 62 des Übersetzungselements 60. In den verbleibenden Zwischenraum zwischen dem Übertragungselement 70 und dem Übersetzungselement 60 greift der halbmondförmige Vorsprung 25 ein.

Das Übertragungselement 70 dreht sich um eine Drehachse, die abgesehen von Toleranzen der Längsachse 1 der Stange 30 entspricht und somit insbesondere der Drehachse des Betätigungselements 50 entspricht.

Die Durchgangsöffnung 74 des Übertragungselements 70 weist einen unrunden Querschnitt auf, vorliegend insbesondere in Form eines Innensechskants, wobei die Kontur der Durchgangsöffnung 74 im Wesentlichen formschlüssig mit der Kontur des Abschnitts 35 der Stange 30 in Eingriff steht.

Eine radiale Lagerung des Übertragungselements 70 erfolgt über einen sich an die Durchgangsöffnung 74 anschließenden, sich in axialer Richtung erstreckenden Steg 76, welcher einen dem Querschnitt der Durchgangsöffnung entsprechenden Innenquerschnitt und eine im wesentlichen zylindrische Außenfläche aufweist und sich in das Lumen des proximalen Rohrabschnitts 24 erstreckt und mit der zylindrischen Außenfläche an der Innenwandung des proximalen Rohrabschnitts 24 gelagert ist.

Die Stange 30 ist durch die Durchgangsöffnung 74 des Übertragungselements 70 geführt. Über den formschlüssigen Eingriff der Durchgangsöffnung 74 und dem Abschnitt 35 der Stange 30 ist eine drehfeste Verbindung zwischen dem Übertragungselement 70 und der Stange 30 gewährleistet. Alternativ kann das Übertragungselement 70 auch einstückig mit der Stange 30 verbunden sein. Eine lösbare Verbindung zwischen der Stange 30 und dem Übertragungselement 70 ermöglicht jedoch eine bessere Reinigung des Instruments, da die Stange 30 aus dem Rohr 20 herausgezogen werden kann.

Wird das Betätigungselement 50 gedreht, dreht sich sowohl das Übersetzungselement 60 als auch das Übertragungselement 70 und somit die Stange 30 mit gleichem Drehsinn.

Das proximale Ende 32 der Stange 30 verläuft innerhalb des Rohres 20 und ragt insbesondere nicht über das proximale Ende 22 des Rohres 20, d.h. insbesondere das zweite Ende 24b des proximalen Rohrabschnitts 24 des Rohres 20, hinaus. Das proximale Ende 22 des Rohres 20 kann durch eine Abschlusskappe oder auch durch einen Handgriff 29 abgedeckt sein. Dies hat den Vorteil, dass bei Schlägen, bspw. mit einem Hammer, auf das proximale Ende des Instruments 10 die Schläge auf das proximale Ende 22 des Rohres 20 erfolgen und somit die Kraft nicht direkt auf die Stange 30 und insbesondere das Arbeitsende 23 übertragen wird.

Die Verstellvorrichtung 40 ermöglicht auf einfache Art und Weise die Initiierung der Drehbewegung der Stange 30 um die Längsachse 1 der Stange 30, ohne dass die Dimensionen des Instruments 10 wesentlich vergrößert werden. Insbesondere wird die Verstellvorrichtung auf einfache Art und Weise zwischen dem distalen Ende 21 und dem proximalen Ende 22, insbesondere zwischen dem distalen Rohrabschnitt 23 und dem proximalen Rohrabschnitt 24, angeordnet.

In dem vorliegenden Ausführungsbeispiel weist die Innenverzahnung 52 des Betätigungselements 50 32 Zähne, die Außenverzahnung des Übersetzungselements 60 25 Zähne, die Innenverzahnung 62 des Übersetzungselements 60 19 Zähne und die Außenverzahnung 72 des Übertragungselements 70 12 Zähne auf. Insbesondere wird dadurch ein Übersetzungsverhältnis von 1:2 erreicht, d.h. bei einer Umdrehung des Betätigungselements 50 macht das Übertragungselement 70 zwei Umdrehungen.

### Bezugszeichenliste

- 10: Instrument

- 20: Rohr
- 21: distales Ende
- 22: proximales Ende
- 23: distaler Rohrabschnitt
- 23a: erstes Ende
- 23b: zweites Ende
- 23c: Nut
- 24: proximaler Rohrabschnitt
- 24a: erstes Ende
- 24b: zweites Ende
- 25: Vorsprung
- 26: Vorsprung
- 27: Schraube
- 28: Stift
- 29: Handgriff

- 30: Stange
- 31: distales Ende
- 32: proximales Ende
- 33: Arbeitsende
- 34: Gewinde
- 35: Abschnitt

- 40: Verstellvorrichtung
- 50: Betätigungselement
- 52: Innenverzahnung
- 54: Profil
- 60: Übersetzungselement
- 62: Innenverzahnung
- 64: Außenverzahnung
- 66: Steg

- 70: Übertragungselement
- 72: Außenverzahnung
- 74: Durchgangsöffnung
- 76: Steg

- 1: Längsachse
- d: Drehachse

## Patentansprüche

1. Chirurgisches Instrument (10), insbesondere zum Halten eines Implantats, mit einem Rohr (20), welches ein distales Ende (21) und ein proximales Ende (22) aufweist, und einer koaxial in dem Rohr (20) angeordneten Stange (30), welche ein distales (31) und ein proximales Ende (32) aufweist, wobei an dem distalen Ende (31) der Stange (30) ein Arbeitsende (33) angeordnet ist, und wobei die Stange (30) mittels einer Verstellvorrichtung (40) um ihre Längsachse (1) drehbar in dem Rohr (20) angeordnet ist, wobei das proximale Ende (32) der Stange (30) innerhalb des Rohres (20) angeordnet ist,
**dadurch gekennzeichnet, dass** die Verstellvorrichtung (40) ein Betätigungselement (50) aufweist, welches als Zahnradring mit einer Innenverzahnung (52) ausgebildet ist, wobei innerhalb des Betätigungselements (50) ein Übersetzungselement (60) angeordnet ist, welches als Zahnradring mit einer Außenverzahnung (64), welche mit der Innenverzahnung (52) des Betätigungselements (50) in Eingriff steht, und einer Innenverzahnung (62) ausgebildet ist, und wobei innerhalb des Übersetzungselements (60) ein Übertragungselement (70) angeordnet ist, welches eine Außenverzahnung (72) aufweist, welche mit der Innenverzahnung (62) des Übersetzungselements (60) in Eingriff steht.

2. Chirurgisches Instrument nach Anspruch 1,
**dadurch gekennzeichnet, dass** die Verstellvorrichtung (40) zwischen dem distalen (21) und dem proximalen Ende (22) des Rohres (20) angeordnet ist.

3. Chirurgisches Instrument nach Anspruch 1 oder 2,
**dadurch gekennzeichnet, dass** das Rohr (20) einen distalen Rohrabschnitt (23) und einen proximalen Rohrabschnitt (24) aufweist und die Verstellvorrichtung 40) zwischen dem distalen Rohrabschnitt (23) und dem proximalen Rohrabschnitt (24) angeordnet ist.

4. Chirurgisches Instrument nach einem der vorhergehenden Ansprüche,
**dadurch gekennzeichnet, dass** an dem Rohr (20) ein Handgriff (29) angeordnet ist.

5. Chirurgisches Instrument nach Anspruch 4,
**dadurch gekennzeichnet, dass** die Verstellvorrichtung (40) axial anschließend an das distale Ende des Handgriffs (29) angeordnet ist.

6. Chirurgisches Instrument nach einem der vorhergehenden Ansprüche,
**dadurch gekennzeichnet, dass** die Verstellvorrichtung (40) als Zahnradgetriebe ausgebildet ist.

7. Chirurgisches Instrument nach einem der vorhergehenden Ansprüche,
**dadurch gekennzeichnet, dass** die Verstellvorrichtung (40) als Planetengetriebe ausgebildet ist.

8. Chirurgisches Instrument nach einem der vorhergehenden Ansprüche,
**dadurch gekennzeichnet, dass** die Verstellvorrichtung (40) ein Betätigungselement (50) aufweist, wobei das Betätigungselement (50) und die Stange (30) gleiche Drehrichtung aufweisen.

9. Chirurgisches Instrument nach einem der vorhergehenden Ansprüche,
**dadurch gekennzeichnet, dass** die Drehachse des Betätigungselements (50) und die Drehachse des Übertragungselements (70) identisch sind, während die Drehachse des Übersetzungselements (60) parallel versetzt zu den Drehachsen des Betätigungselements (50) und des Übertragungselements (70) angeordnet ist.

10. Chirurgisches Instrument nach einem der vorhergehenden Ansprüche,
**dadurch gekennzeichnet, dass** das Übertragungselement(70) mit der Stange (30) drehfest verbunden ist.

11. Chirurgisches Instrument nach einem der vorhergehenden Ansprüche ,
**dadurch gekennzeichnet, dass** das Übertragungselement (70) eine Durchgangsöffnung (74) mit einer unrunden Innenkontur aufweist, in welche die Stange (30) mit einem Abschnitt (35), der eine entsprechende Außenkontur aufweist, im wesentlichen formschlüssig einsetzbar ist.

12. Chirurgisches Instrument nach einem der vorhergehenden Ansprüche,
**dadurch gekennzeichnet, dass** der Außendurchmesser der Verstellvorrichtung (40), insbesondere des Betätigungselements (50), im Wesentlichen dem Außendurchmesser des Rohres (20) und/oder des Handgriffs (29) entspricht.

13. Chirurgisches Instrument nach einem der vorhergehenden Ansprüche,
**dadurch gekennzeichnet, dass** das distale Ende (31) der Stange (30) als Gewinde (34), Sechskant, Sechsrund, Zahnrad, Kegelrad oder Schnecke ausgebildet ist.

## Claims

1. Surgical instrument (10), in particular for holding an implant, comprising a tube (20) which has a distal end (21) and a proximal end (22), and a rod (30) which is arranged coaxially in the tube (20) and has a distal end (31) and a proximal end (32), wherein a working end (33) is arranged at the distal end (31) of the rod (30), and wherein the rod (30) is arranged in the tube (20) in such a way as to be rotatable about its longitudinal axis (1) by means of an adjustment device (40), wherein the proximal end (32) of the rod (30) is arranged inside the tube (20), **characterised in that** the adjustment device (40) has an actuating element (50) which is designed as a toothed wheel ring having an inner toothing (52), wherein arranged inside the actuating element (50) is a step-up element (60) which is designed as a toothed wheel ring having an outer toothing (64), which meshes with the inner toothing (52) of the actuating element (50), and an inner toothing (62), and wherein arranged inside the step-up element (60) is a transmission element (70) which has an outer toothing (72) that meshes with the inner toothing (62) of the step-up element (60).

2. Surgical instrument according to claim 1, **characterised in that** the adjustment device (40) is arranged between the distal end (21) and the proximal end (22) of the tube (20).

3. Surgical instrument according to claim 1 or 2, **characterised in that** the tube (20) has a distal tube section (23) and a proximal tube section (24) and the adjustment device (40) is arranged between the distal tube section (23) and the proximal tube section (24).

4. Surgical instrument according to one of the preceding claims, **characterised in that** a handle (29) is arranged on the tube (20).

5. Surgical instrument according to claim 4, **characterised in that** the adjustment device (40) is arranged axially next to the distal end of the handle (29).

6. Surgical instrument according to one of the preceding claims, **characterised in that** the adjustment device (40) is designed as a toothed wheel gear mechanism.

7. Surgical instrument according to one of the preceding claims, **characterised in that** the adjustment device (40) is designed as a planetary gear mechanism.

8. Surgical instrument according to one of the preceding claims, **characterised in that** the adjustment device (40) has an actuating element (50), wherein the actuating element (50) and the rod (30) have the same direction of rotation.

9. Surgical instrument according to one of the preceding claims, **characterised in that** the axis of rotation of the actuating element (50) and the axis of rotation of the transmission element (70) are identical, while the axis of rotation of the gear ratio element (60) is arranged parallel to and offset from the axes of rotation of the actuating element (50) and of the transmission element (70).

10. Surgical instrument according to one of the preceding claims, **characterised in that** the transmission element (70) is connected to the rod (30) so as to rotate therewith.

11. Surgical instrument according to one of the preceding claims, **characterised in that** the transmission element (70) has a through-opening (74) with a non-round inner contour, into which the rod (30) can be inserted in a substantially form-fitting manner with a section (35) that has a corresponding outer contour.

12. Surgical instrument according to one of the preceding claims, **characterised in that** the external diameter of the adjustment device (40), in particular of the actuating element (50), substantially corresponds to the external diameter of the tube (20) and/or of the handle (29).

13. Surgical instrument according to one of the preceding claims, **characterised in that** the distal end (31) of the rod (30) is designed as a thread (34), hexagon, hexalobe, toothed wheel, bevel gear or worm.

## Revendications

1. Instrument chirurgical (10) en particulier pour maintenir un implant comportant un tube (20) comprenant une extrémité distale (21) et une extrémité proximale (22) ainsi qu'une tige (30) montée coaxialement dans le tube (20) et comprenant une extrémité distale (31) et une extrémité proximale (32), une extrémité de travail (33) étant montée à l'extrémité distale (31) de la tige (30), et la tige (30) étant montée au moyen d'un dispositif de réglage (40) mobile en rotation dans le tube (20) autour de son axe longitudinal (1), l'extrémité proximale (32) de la tige (30) étant située à l'intérieur du tube (20),
caractérisé en que
le dispositif de réglage (40) comporte un élément d'actionnement (50) qui est réalisé sous la forme d'une bague dentée comportant une denture interne (52), à la partie interne de l'élément d'actionnement (50) étant monté un élément de démultiplication (60) réalisé sous la forme d'une bague dentée comportant une denture externe (64) venant en prise avec la denture interne (52) de l'élément d'actionnement (50), et une denture interne (62), et, à la partie interne de l'élément de démultiplication (60) étant monté un élément de transmission (70) comportant une denture externe (72) venant en prise avec la denture interne (62) de l'élément de démultiplication (60).

2. Instrument chirurgical conforme à la revendication 1,
caractérisé en que
le dispositif de réglage (40) est monté entre l'extrémité distale (21) et l'extrémité proximale (22) du tube (20).

3. Instrument chirurgical conforme à la revendication 1 ou 2, caractérisé en que
le tube (20) comporte un tronçon tubulaire distal (23) et un tronçon tubulaire proximal (24) et le dispositif de réglage (40) est monté entre le tronçon tubulaire distal (23) et me tronçon tubulaire proximal (24),

4. Instrument chirurgical conforme à l'une des revendications précédents,
caractérisé en qu'
une poignée (29) est montée sur le tube (20).

5. Instrument chirurgical conforme à la revendication 4,
caractérisé en que
le dispositif de réglage (40) est monté axialement directement contigu à l'extrémité distale de la poignée (29).

6. Instrument chirurgical conforme à l'une des revendications précédentes,
caractérisé en que
le dispositif de réglage (40) est réalisé sous la forme d'une transmission par engrenage.

7. Instrument chirurgical conforme à l'une des revendications précédentes,
caractérisé en que
le dispositif de réglage (40) est réalisé sous la forme d'un engrenage planétaire.

8. Instrument chirurgical conforme à l'une des revendications précédentes,
caractérisé en que
le dispositif de réglage (40) comporte un élément d'actionnement (50), l'élément d'actionnement (50) et la tige (30) ayant le même sens de rotation.

9. Instrument chirurgical conforme à l'une des revendications précédentes,
caractérisé en que
l'axe de rotation de l'élément d'actionnement (50) et l'axe de rotation de l'élément de transmission (70) sont identiques alors que l'axe de rotation de l'élément de démultiplication (60) est décalé parallèlement par
rapport aux axes de rotation de l'élément d'actionnement (50) et de l'élément de transmission (70).

10. Instrument chirurgical conforme à l'une des revendications précédentes,
caractérisé en que
l'élément de transmission (70) est relié solidairement en rotation à la tige (30).

11. Instrument chirurgical conforme à l'une des revendications précédentes,
caractérisé en que
l'élément de transmission (70) comporte une ouverture traversante (74) ayant un contour interne non circulaire dans laquelle la tige (30) peut être introduite essentiellement par une liaison par la forme avec un tronçon (35) présentant un contour externe correspondant,

12. Instrument chirurgical conforme à l'une des revendications précédentes,
caractérisé en que
le diamètre externe du dispositif de réglage (40) en particulier de l'élément d'actionnement (50) correspond essentiellement au diamètre externe du tube (20) et/ou de la poignée (29).

13. Instrument chirurgical conforme à l'une des revendications précédentes,
caractérisé en que
l'extrémité distale (31) de la tige (30) est réalisée sous la forme d'un filetage (34), d'une tête hexagonale, d'une rondelle à six pans, d'une roue dentée, d'une roue conique ou d'une vis sans fin.
